# EUROPEAN PATENT APPLICATION

(11) **EP 4 748 333 A1**
(43) Date of publication of application: **27.05.2026**
(21) Application number: 24848187.1
(22) Date of filing: 26.07.2024
(51) Int. Cl.: A61B 34/20

(54) **TRACKING METHOD AND APPARATUS, MEDICAL ASSISTANCE SYSTEM, MEDIUM, AND COMPUTING DEVICE**

(30) Priority: 28.07.2023 CN 202310950151; 28.07.2023 CN 202310947864; 28.07.2023 CN 202310947854
(71) Applicant: Cornerstone Technology (Shenzhen) Limited, Shenzhen, Guandong 518066 (CN)
(72) Inventor: LEUNG, Cheuk Hei, Shenzhen, Guangdong 518066 (CN); KWOK, Derek Hang Chun, Shenzhen, Guangdong 518066 (CN)
(74) Representative: Balder IP Law, S.L.
(86) International application number: PCT/CN2024/107910
(87) International publication number: WO 2025/026230

(57) **Abstract**

A tracking method, an apparatus, a medical assistance system, a medium and a computing device. The method includes: acquiring a video frame of a first target object and initial pose information indicative of an initial pose of the first target object when the video frame is captured (S61); acquiring a three-dimensional model of the target object (S62); acquiring prediction pose information of the first target object through a pre-trained neural network based on the video frame (S63); acquiring detection pose information of the first target object based on the initial pose information and the three-dimensional model (S64); matching the prediction pose information and the detection pose information to obtain a matching result (S65); and tracking the first target object according to the matching result (S66).

## Description

### CROSS-REFERENCE TO RELATED APPLICATIONS

The present application claims priority to Chinese Patent Application No. 202310950151.2, filed on July 28, 2023, entitled "METHOD FOR TRACKING TARGET OBJECT, APPARATUS AND SYSTEM, MEDIUM AND COMPUTING DEVICE", Chinese Patent Application No. 202310947864.3, filed on July 28, 2023, entitled "METHOD FOR GENERATING LABELS FOR IMAGES, APPARATUS AND SYSTEM, MEDIUM AND COMPUTING DEVICE", and Chinese Patent Application No. 202310947854.X, filed on July 28, 2023, entitled "METHOD FOR GENERATING LABELS FOR IMAGES, APPARATUS AND SYSTEM, MEDIUM AND COMPUTING DEVICE", the entire contents of which are incorporated herein by reference.

### TECHNICAL FIELD

The present application relates to a field of image processing technology, and in particular, to a method for tracking a target object, an apparatus and a system, a medium and a computing device.

### BACKGROUND

During a surgical procedure performed by a surgical robot system, it is generally desirable to obtain accurate pose information of surgical instruments to achieve real-time tracking of the surgical instruments. This is particularly advantageous when the surgical instruments are outside a field of view of an endoscopic camera or are occluded within the field of view of the endoscopic. Related technologies tracks a surgical instrument typically based on pose information of the surgical instrument determined with measurement results from sensors. However, due to errors in the measurements of the sensors and a cumulative nature of these errors, a tracking accuracy of the aforementioned method is relatively low.

On the other hand, in related technologies, labels for a target object are generally obtained based on measurement results from sensors. Due to errors in the sensor measurements, the obtained labels are inaccurate. A neural network may be employed in the tracking of the target object, and training of the neural network relies on a large number of labeled images. The neural network, when trained with inaccurate labels of the images, will work inaccurately, leading to inaccurate tracking results for the target object.

### SUMMARY

In a first aspect, embodiments of the present application provide a tracking method. The method includes: acquiring a video frame of a first target object and initial pose information indicative of an initial pose of the first target object when the video frame is captured; acquiring a three-dimensional model of the first target object; acquiring prediction pose information of the first target object through a pre-trained neural network based on the video frame; acquiring detection pose information of the first target object based on the initial pose information and the three-dimensional model; matching the prediction pose information and the detection pose information to obtain a matching result; and tracking the first target object according to the matching result.

In some embodiments, the prediction pose information includes a prediction pixel region, prediction keypoint information, and prediction orientation information of the first target object, and the detection pose information includes a detection pixel region, detection keypoint information, and detection orientation information of the first target object; the matching the prediction pose information and the detection pose information includes: matching the prediction pixel region and the detection pixel region, matching the prediction keypoint information and the detection keypoint information, and matching the prediction orientation information and the detection orientation information.

In some embodiments, the matching result includes a pixel region matching result, a keypoint matching result, and an orientation information matching result, the tracking the first target object according to the matching result includes: determining a first confidence score that the prediction pixel region matches the detection pixel region according to the pixel region matching result; determining a second confidence score that the prediction keypoint information matches the detection keypoint information according to the keypoint matching result; determining a third confidence score that the prediction orientation information matches the detection orientation information according to the orientation information matching result; and tracking the first target object according to the first confidence score, the second confidence score, and the third confidence score.

In some embodiments, the acquiring detection pose information of the first target object based on the initial pose information and the three-dimensional model includes: projecting the three-dimensional model into a coordinate system of an image capturing device according to the initial pose to obatin the detection keypoint information and the detection orientation information of the first target object in the coordinate system of the image capturing device, wherein the image capturing device is configured to capture the video frame; and projecting the three-dimensional model onto a two-dimensional image plane corresponding to the video frame according to the initial pose to obtain the detection pixel region of the first target object within the two-dimensional image plane.

In some embodiments, the acquiring prediction pose information of the first target object through the pre-trained neural network includes: acquiring a bounding box of the first target object in the video frame; and acquiring the prediction pose information of the first target object in the video frame based on the bounding box of the first target object in the video frame.

In some embodiments, the neural network includes a plurality of feature extraction layers configured to perform feature extraction on the target video frame; the bounding box of the first target object in the video frame is acquired based on features output from at least one first feature extraction layer among the plurality of feature extraction layers; the prediction pose information of the first target object in the video frame is acquired based on features output from at least one second feature extraction layer among the plurality of feature extraction layers; wherein each second feature extraction layer is prior to each first feature extraction layer.

In some embodiments, each of the feature extraction layers includes an encoder and a decoder; an output end of an encoder of an i-th feature extraction layer is connected to an input end of an encoder of an (i+1)-th feature extraction layer and an input end of a decoder of the i-th feature extraction layer; an input end of the decoder of the i-th feature extraction layer is connected to an output end of a decoder of the (i+1)-th feature extraction layer; wherein i is a positive integer; wherein each encoder is configured to perform down-sampling processing on features input the encoder, and each decoder is configured to perform up-sampling processing on features input the decoder.

In some embodiments, the acquiring the bounding box of the first target object in the video frame includes: acquiring the bounding box of the first target object in the video frame based on the video frame and a tracking result of the first target object in a prior video frame.

In some embodiments, the acquiring the prediction pose information of the first target object in the video frame based on the bounding box of the first target object in the video frame includes: performing pooling processing on features within the bounding box of the first target object in the video frame to obtain the prediction pose information of the first target object.

In some embodiments, the neural network is pre-trained based on sample images and label information of the sample images.

In some embodiments, the sample images and the label information of the sample images are generated by: acquiring a first image of a second target object and initial pose information indicative of an initial pose of the second target object when the first image is captured; acquiring a three-dimensional model of the second target object; projecting the three-dimensional model of the second target object onto the first image based on the initial pose information of the second target object to obtain a projected pixel region; determining corrected pose information based on the initial pose information of the second target object and an overlap degree between the projected pixel region and a target pixel region of the second target object on the first image; generating a second image of the second target object based on the first image, wherein the second image is regarded as one of the sample images; and generating label information of the second target object in the second image based on the corrected pose information.

In some embodiments, the first image is captured when the second target object is against a first preset background.

In some embodiments, the generating the second image of the second target object based on the first image includes: replacing the first preset background in the first image with a second preset background to obtain the second image.

In some embodiments, the label information of the sample images is generated by a further operation including: performing post-processing on the second image; the post-processing includes at least one of: blurring processing, sharpening processing, noise reduction processing, and enhancement processing.

In some embodiments, the corrected pose information is indicative of a pose of the second target object when the overlap degree is maximized.

In some embodiments, the determining corrected pose information based on the initial pose information and the overlap degree between the projected pixel region and the target pixel region of the second target object on the first image, includes: optimizing the initial pose information using a preset pose optimization algorithm, and recalculating the overlap degree between the projected pixel region and the target pixel region; and determining the corrected pose information in response to the overlap degree between the projected pixel region and the target pixel region being maximum.

In some embodiments, the overlap degree between the projected pixel region and the target pixel region is determined based on an intersection over union (IoU), a generalized intersection over union (GIoU), or a dice loss between the projected pixel region and the target pixel region.

In some embodiments, the label information of the sample images is generated by further operations including: acquiring a mask of the second target object in the first image; and determining the overlap degree between the mask of the second target object and the projected pixel region.

In some embodiments, before determining the overlap degree between the mask of the second target object and the projected pixel region, the method further includes: performing smoothing processing on the mask.

In some embodiments, the first target object or the second target object includes at least one surgical instrument; each surgical instrument is held by a robotic arm of a surgical robot, and the robotic arm is provided with one or more sensor configured to detect the initial pose of the surgical instrument held by the robotic arm; the video frame and the first image are captured by an image capturing device.

In some embodiments, the three-dimensional model of the surgical instrument is associated with type and model information of the surgical instrument; the acquiring the three-dimensional model of the first target object or the second target object includes: acquiring a three-dimensional model of the surgical instrument held by the robotic arm according to the type and model information of the surgical instrument held by the robotic arm.

In some embodiments, the method further includes: determining the type model information of the surgical instrument held by each robotic arm based on an operation log of the surgical robot; or determining the type model information of the surgical instrument held by each robotic arm based on user input.

In some embodiments, the label information includes the corrected pose information, and the type and model information of the surgical instrument.

In a second aspect, the present application provides a tracking apparatus, including: a first acquisition module configured to acquire a video frame of a first target object and initial pose information indicative of an initial pose of the first target object when the video frame is captured, and to acquire a three-dimensional model of the first target object; a second acquisition module configured to acquire prediction pose information of the first target object through a pre-trained neural network based on the video frame; a third acquisition module configured to acquire detection pose information of the first target object based on the initial pose information of the first target object and the three-dimensional model; a matching module configured to match the prediction pose information and the detection pose information to obtain a matching result; and a tracking module configured to track the first target object according the matching result.

In some embodiments, the tracking apparatus further includes: a fourth acquisition module configured to acquire a first image of a second target object and initial pose information indicative of an initial pose of the second target object when the first image is captured, and to acquire a three-dimensional model of the second target object; a projection module configured to project the three-dimensional model of the second target object onto the first image based on the initial pose information of the second target object to obtain a projected pixel region; a correction module configured to determine corrected pose information based on the initial pose information and an overlap degree between the projected pixel region and a target pixel region of the second target object on the first image; a generation module configured to generate a second image of the second target object based on the first image; and a determination module configured to determine label information of the second target object in the second image based on the corrected pose information.

In a third aspect, the present application provides a computer-readable storage medium having a computer program stored thereon, which when executed by a processor causes the processor to implement the method according to any one of the embodiments.

In a fourth aspect, the present application provides a computing device, including a memory, a processor, and a computer program stored on the memory and executable by the processor, wherein the processor, when executing the program, implements the method according to any one of the embodiments.

In a fifth aspect, the present application provides a medical assistance system, wherein the system includes: an image capturing device configured to capture a video frame of a first target object; a pose sensing device configured to detect an initial pose of the first target object when the video frame is captured; and the computing device according to the fourth aspect having a pre-trained neural network deployed thereon.

In some embodiments, the image capturing device is further configured to capture a first image of a second target object; the pose sensing device is further configured to detect an initial pose of the second target object when the first image is captured.

In some embodiments, the first target object or the second target object is a surgical instrument; the system further includes: a surgical robot including at least one robotic arm, each robotic arm being configured to hold a surgical instrument and provided with a pose sensing device.

In some embodiments, the first target object or the second target object is a surgical instrument; the system further includes: a surgical robot including at least two robotic arms, the image capturing device and the surgical instrument each being held by a respective one of the at least two robotic arms, and at least the respective one of the at least two robotic arms which holds the surgical instrument being provided with the pose sensing device.

It should be understood that the foregoing general description and the following detailed description are exemplary and explanatory only, and are not restrictive of the present disclosure.

### BRIEF DESCRIPTION OF THE DRAWINGS

The accompanying drawings, which are incorporated in and constitute a part of this specification, illustrate embodiments consistent with the present disclosure and, together with the description, serve to explain the technical solutions of the present disclosure.
FIG. 1 is a schematic diagram of a robot surgical system according to an embodiment of the present application.
FIG. 2 is a schematic diagram of a patient side robot according to an embodiment of the present application.
FIG. 3 is a flowchart of a method for generating label information for images according to an embodiment of the present application.
FIG. 4 is an overall flowchart of the method shown in FIG. 3.
FIG. 5 is a schematic diagram of images in a processing flow shown in FIG. 4.
FIG. 6 is a flowchart of another method for generating label information for images according to an embodiment of the present application.
FIG. 7 is an overall flowchart of the method shown in FIG. 6.
FIG. 8 is a flowchart of a method for tracking a target object in an image according to an embodiment of the present application.
FIG. 9A is a schematic structural diagram of a neural network according to an embodiment of the present application.
FIG. 9B is a schematic structural diagram of a more specific neural network according to an embodiment of the present application.
FIG. 10A is an overall flowchart of the method shown in FIG. 8.
FIG. 10B is a schematic diagram of a multi-target tracking process.
FIG. 11 is a block diagram of a label generation apparatus according to an embodiment of the present application.
FIG. 12 is a block diagram of another label generation apparatus according to an embodiment of the present application.
FIG. 13 is a block diagram of a tracking apparatus according to an embodiment of the present application.
FIG. 14 is a schematic diagram of a computing device according to an embodiment of the present application.
FIG. 15 is a schematic diagram of a tracking system according to an embodiment of the present application.

### DETAILED DESCRIPTION

Exemplary embodiments will be described in detail here, examples of which are illustrated in the accompanying drawings. When the following description refers to the accompanying drawings, unless otherwise indicated, the same numerals in different drawings denote the same or similar elements. The implementations described in the following exemplary embodiments do not represent all implementations consistent with the present application. Rather, they are merely examples of apparatuses and methods consistent with some aspects of the present application as detailed in the appended claims.

The terms used in the present application are for the purpose of describing particular embodiments only and are not intended to limit the present disclosure. The singular forms "a", "the", and "said" used in the present application and the appended claims are also intended to include plural forms, unless the context clearly indicates otherwise. It should also be understood that the term "and/or" as used herein refers to and includes any and all possible combinations of one or more of the associated listed items. Additionally, the term "at least one" as used herein indicates any one of a plurality or any combination of at least two of a plurality.

It should be understood that although the terms first, second, third, etc. may be used in the present application to describe various information, such information should not be limited by these terms. These terms are only used to distinguish information of the same type from one another. For example, without departing from the scope of the present application, first information may also be referred to as second information, and similarly, second information may also be referred to as first information. Depending on the context, the word "if" as used herein may be interpreted as "when", "upon", or "in response to determining".

To enable those skilled in the art to better understand the technical solutions in the embodiments of the present application, and to make the above-mentioned objectives, features, and advantages of the embodiments of the present application more apparent and easier to understand, the technical solutions in the embodiments of the present application will be further described in detail below with reference to the accompanying drawings.

Before using a neural network Net to track a target object Obj, it is necessary to train the neural network Net using a large number of labeled images. An example is provided below in conjunction with specific application scenarios. It should be understood that the application scenarios described below are for illustrative purposes only and is not intended to limit the present disclosure.

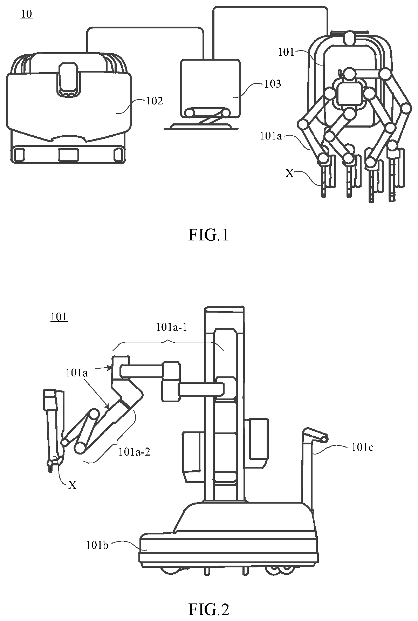

In a surgical scenario, the target object Obj includes surgical instruments X. The surgical instruments X are widely used in various surgeries. A surgeon can manipulate the surgical instrument X through a robot surgical system 10 to perform surgery. FIG. 1 is a schematic diagram of the robot surgical system 10. During surgery, a patient is positioned in front of a patient side robot (PSR) 101. The patient side robot 101 includes one or more robotic arms 101a. An end of each robotic arm 101a is used to hold one or more surgical instruments X. The surgeon can control the robotic arm 101a through a surgeon console (SGC) 102, thereby controlling the surgical instruments X to perform surgical operations on the patient. The robotic arms 101a can also hold an image capturing device (e.g., an endoscopic camera, not shown). The surgeon can control the robotic arm 101a holding the endoscopic camera through the surgeon console 102 to move and maintain the endoscopic camera near the patient's lesion area for capturing surgical scenes including the patient's lesion and surrounding tissues, and the surgical instruments X. During surgery, the surgical instruments X and/or the endoscopic camera on the robotic arms 101a are inserted into the patient's body through a preset orifice on the patient and can rotate around a center point of the orifice (generally referred to as a remote center of motion point, RCM). Images captured by the endoscopic camera are transmitted to a vision cart (VCT) 103 for image processing and recording, and the processed images are displayed on display devices of the vision cart 103 and the surgeon console 102 for observation by the surgeon and other surgical personnel.

During surgery, it is generally desirable to obtain accurate pose information of the surgical instrument to achieve real-time tracking. This is particularly advantageous in a case that the surgical instrument is outside a field of view of the endoscopic camera or is occluded within the field of view of the endoscopic camera. In some cases, a neural network is used to track the surgical instrument. Before deploying a neural network model into practice, the neural network model needs to be trained.

Dataset preparation is as follows.

Training the neural network model requires preparing a large dataset of images of the target object and labels regarding the target object in the images. In the application scenario of the embodiments of the present disclosure, the target object Obj includes the surgical instrument X, and label information may include accurate pose information of the surgical instrument X.

FIG. 2 shows a schematic diagram of the patient side robot 101. As shown in FIG. 2, the patient side robot 101 includes a chassis 101b, a push handle 101c, and at least one robotic arm 101a (for ease of illustration, only one robotic arm 101a is shown in the figure). Each robotic arm 101a includes a set-up arm 101a-1 and an operation arm 101a-2. The robotic arm 101a-2 includes one or more sensors, such as a displacement sensor, an orientation sensor, and/or a position sensor. Kinematic data of the robotic arm 101a and the surgical instrument X held by the robotic arm 101a can be obtained through detection values of these sensors. Kinematic data of the surgical instrument X can be pose information of the surgical instrument X. However, due to errors in sensor measurements and a cumulative nature of transmission errors of the robotic arm 101a, the kinematic data obtained by the sensors includes noise. The pose information of the surgical instrument X generated solely based on the noisy kinematic data mentioned above is relatively inaccurate and cannot be used for training the neural network.

Embodiments of the present application acquires prediction pose information of the target object through the neural network, acquires detection pose information of the target object based on initial pose information indicative of an initial pose the target object and a three-dimensional model of the target object, matches the prediction pose information and the detection pose information, and tracks the target object based on a matching result, thereby effectively improving tracking accuracy. Furthermore, by acquiring a first image of the target object, projecting the three-dimensional model of the target object onto the first image based on the initial pose information of the target object when the first image is captured to obtain a projected pixel region, and then correcting the initial pose information based on an overlap degree between the projected pixel region and a target pixel region, corrected pose information of the target object in the first image with better accuracy is obtained. Label information of the target object in a second image of the target object generated based on the corrected pose information is of better accuracy, thereby improving accuracy of a pre-trained neural network, and further improving accuracy of target object tracking.

### First embodiment.

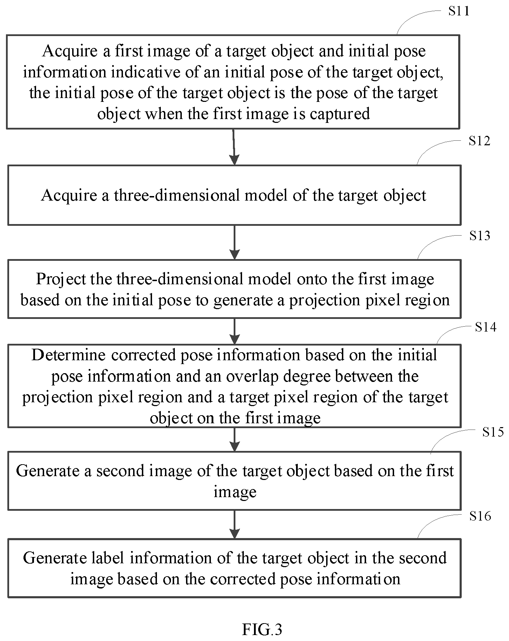

An embodiment of the present application provides a method for generating labels for images. Referring to FIG. 3, the method includes operations as follows.

In operation S11, a first image Img1 of a target object Obj and initial pose information indicative of an initial pose Pose0 of the target object Obj are acquired. The initial pose Pose0 is the pose of the target object Obj at the time when the first image Img1 is captured.

In operation S12, a three-dimensional model Mod of the target object Obj is acquired.

In operation S13, the three-dimensional model Mod is projected onto the first image Img1 based on the initial pose information Pose0 to obtain a projected pixel region Rm.

In operation S14, corrected pose information indicative of a corrected pose Posel is determined based on the initial pose information and an overlap degree between the projected pixel region Rm and a target pixel region Ro of the target object Obj on the first image Img1.

In operation S15, a second image Img2 of the target object Obj is generated based on the first image Img1.

In operation S16, label information of the target object Obj in the second image Img2 is generated based on the corrected pose information indicative of the corrected pose Pose1.

Implementation details of generating labels for images according to the present application are illustrated below with examples.

In operation S11, the target object Obj may be a surgical instrument X. However, it should be understood that in other application scenarios, the target object Obj may also be other objects. For example, in an image surveillance scenario, the target object Obj may be a person or an animal under surveillance; in a traffic scenario, the target object Obj may be a vehicle. For ease of explanation, the solutions of the embodiments of the present disclosure are described below using the surgical scenario shown in FIG. 1 and FIG. 2 as an example.

The first image Img1 of the surgical instrument X can be a real picture captured by an image capturing device. The surgical instrument X includes, but is not limited to, one or more of a scalpel, tissue scissors, surgical forceps, needle holders, vascular clamps, etc. Each surgical instrument X can be held by one robotic arm 101a of a surgical robot. The image capturing device may also be held by a robotic arm 101a of the surgical robot, mounted on a stand, or fixed at other locations (e.g., wall or table). A pose sensing device may be provided on the robotic arm 101a holding the surgical instrument X to detect an initial pose Pose0 of the surgical instrument X to obtain initial pose information, the initial pose Pose0 of the surgical instrument X is a pose of the surgical instrument X at a time when the first image Img1 is captured. In some embodiments, the robotic arm 101a includes a plurality of links sequentially connected, with adjacent links connected via rotary joints. The surgical instrument X is mounted on a distal link among the links. The pose sensing device may include encoders for the rotary joints, displacement sensors for a linear drive module on the distal link, encoders for a tool drive module on the distal link, etc. Due to errors of the pose sensing device and other reasons, the initial pose information indicative of the initial pose Pose0 is noisy and cannot accurately reflect a true pose of the surgical instrument. In some embodiments, the robotic arm 101a holding the image capturing device may also be provided with a pose sensing device to detect the pose of the image capturing device.

In some embodiments, the first image Img1 may be captured when the surgical instrument X is against a first preset background. A pixel value difference between pixel values of the first preset background and pixel values of the surgical instrument X may be greater than a preset value. For example, if a color of the surgical instrument X is white, the first preset background may be black. Furthermore, the first preset background may also be a pure color background (i.e., including only one color), and the first preset background may have little texture. This can reduce interference from the color and texture of the first preset background on subsequent processing of the first image Img1, thereby improving the accuracy of the acquired label information. Alternatively, image acquisition may be performed on the surgical instrument X in practice (e.g., during surgery) to obtain the first image Img1.

In operation S12, a three-dimensional model Mod of the surgical instrument X can be acquired. A surgical instrument X of a determined model in a determined type has a pre-determined three-dimensional model Mod. In some embodiments, surgical instruments can be classified into the following types based on their functions, such as scalpels, tissue scissors, surgical forceps, vascular clamps, etc. For each type of surgical instrument, it can be divided into different models based on its structure, dimensions, and other characteristics. The three-dimensional model Mod of the surgical instrument X is established when its design is completed or before production. The present disclosure does not specifically limit the method for establishing the three-dimensional model Mod. The three-dimensional model Mod of the surgical instrument X may be pre-stored in a storage device. Accordingly, based on the type and model information of the surgical instrument X held by the robotic arm 101a, the three-dimensional model Mod of the surgical instrument X held by that robotic arm 101a can be retrieved from the storage device. In some embodiments, if the surgical instrument X held by a No. 1 robotic arm 101a is a No. 10 scalpel, the retrieved three-dimensional model Mod is a three-dimensional model Mod corresponding to the No. 10 scalpel; if the surgical instrument X held by a No. 2 robotic arm 101a is a straight vascular clamp, the retrieved three-dimensional model Mod is a three-dimensional model Mod corresponding to the straight vascular clamp.

In some embodiments, the surgical robot can automatically identify the type and model information of the surgical instrument X held by each robotic arm 101a of the patient side robot 101 and record them in a corresponding operation log. The type and model information of the surgical instrument X held by each robotic arm 101a can be retrieved from the operation log of the surgical robot. In other embodiments, the type and model information of the surgical instrument X held by each robotic arm 101a can be determined based on user input. In some embodiments, a surgeon can manually input the type and model information of the surgical instrument X held by each robotic arm 101a on an input interface of the surgeon console 102.

In some embodiments, a correspondence between the type and model information of surgical instruments X and their three-dimensional models Mod can be established in advance. In response to receiving the type and model information of the surgical instrument X held by each robotic arm 101a, the corresponding three-dimensional model Mod of the surgical instrument X can be retrieved based on the aforementioned correspondence. Through the above method, the corresponding three-dimensional model Mod of the surgical instrument X can be retrieved automatically, reducing manual operations and lowering labor costs.

In operation S13, the three-dimensional model Mod may be a three-dimensional model in a physical coordinate system. For example, it may be a standard model located at an origin of a physical coordinate system with a specified posture. In a case that the target object is the surgical instrument X, the initial pose Pose0 of the surgical instrument X may be detected by encoders on the robotic arm where the surgical instrument X is located. The pose information may be obtained based on the pose of the surgical instrument X in a physical coordinate system such as a PSR base coordinate system or a world coordinate system. With the initial pose information, the three-dimensional model Mod can be projected onto the first image Imgl in its initial pose Pose0. In some embodiments, a transformation matrix of the image capturing device can be acquired. This transformation matrix can be obtained by calibrating the image capturing device and is used to achieve conversion between a physical coordinate system (such as the aforementioned PSR base coordinate system) and a coordinate system of the image capturing device. Based on the transformation matrix of the image capturing device, the three-dimensional model Mod can be projected onto the first image Img1.

Ideally, the projected pixel region Rm of the three-dimensional model Mod on the first image Img1 completely coincides with the target pixel region Ro of the target object Obj on the first image Img1. However, due to certain errors in the initial pose Pose0, in reality, the two are not completely overlapped, and the error of the initial pose Pose0 is inversely correlated to some extent with an overlap degree between the projected pixel region Rm and the target pixel region Ro. Therefore, in operation S14, the initial pose Pose0 can be corrected based on the overlap degree between the projected pixel region Rm and the target pixel region Ro.

The overlap degree between the projected pixel region Rm and the target pixel region Ro can be determined based on an intersection over union (IoU), a Generalized Intersection over Union (GIoU), a dice loss, or other parameters that can characterize the overlap degree between the projected pixel region Rm and the target pixel region Ro.

In some embodiments, a mask of the target object Obj in the first image Img1 can be extracted, and the overlap degree between the projected pixel region Rm and the target pixel region Ro can be determined based on the mask of the target object Obj and the projected pixel region Rm. The mask of the target object Obj in the first image Img1 can be obtained by performing image processing on the first image Img1 to remove a background region in the first image Img1. Alternatively, the mask of the target object Obj in the first image Img1 can also be obtained through manual annotation. In some embodiments, in a case that the first image Img1 captured when the surgical instrument X is against a first preset background, since the first preset background generally differs significantly from the target object Obj, the mask can be automatically acquired through image processing. In a case where the image acquisition is performed on the surgical instrument X in practice to obtain the first image Img1, the mask can be obtained through manual annotation to reduce influence of a complex background and improve accuracy of mask extraction. By acquiring the mask, the influence of the background region on calculation of the overlap degree can be reduced, thereby improving the accuracy of the calculated overlap degree and further improving the accuracy of the acquired label information. In some embodiments, before determining the overlap degree between the projected pixel region Rm and the target pixel region Ro based on the mask of the target object Obj and the projected pixel region Rm, smoothing processing may also be performed on the mask. Performing smoothing processing can reduce influence of random noise and eliminate abnormal pixels, thereby improving the accuracy and reliability of the acquired mask.

In some embodiments, the corrected pose information is indicative of a pose of the target object Obj when the overlap degree is maximized. Specifically, the corrected pose information indicative of the corrected pose Pose1 can be obtained by, after optimizing the initial pose information indicative of the initial pose Pose0 using a preset pose optimization algorithm, recalculating the overlap degree between the projected pixel region Rm and the target pixel region Ro, and determining the corrected pose information indicative of the corrected pose Pose1 in response to the overlap degree between the projected pixel region Rm and the target pixel region Ro being maximum. The pose optimization algorithm may be a gradient-based optimization algorithm or other global optimization algorithms. Several iterations of optimization may be used to determine the corrected pose information indicative of the corrected pose Pose1. In a first iteration, the overlap degree between the projected pixel region Rm and the target pixel region Ro corresponding to the initial pose Pose0 is determined, and the initial pose information indicative of the initial pose Pose0 is optimized to obtain pose information after the first iteration optimization. In the second iteration optimization, the overlap degree between the projected pixel region Rm and the target pixel region Ro corresponding to the pose after the first iteration optimization is determined, and the pose information after the first iteration optimization is optimized to obtain pose information after the second iteration optimization. This process continues until a preset iteration termination condition is met, for example, a number of iterations reaches a preset threshold, an algorithm execution time reaches a preset duration threshold, or a maximum overlap degree obtained during iteration reaches a preset overlap threshold or the overlap degree obtained during iteration reaches a local maximum, etc. Therefore, the corrected pose Pose1 can be considered as a true pose of the target object Obj.

In operation S15, the second image Img2 of the target object Obj can be generated based on the first image Img1. For example, the first preset background in the first image Img1 can be replaced with a second preset background to obtain the second image Img2. The second preset background is typically obtained by an image acquisition device, such as an endoscope, capturing an image of human tissues. Further, before replacing with the second preset background, operations such as brightness adjustment, orientation transformation, and/or scale transformation may also be performed on the target object Obj in the first image Img1. The second preset background may be related to the application scenario. For example, in the surgical scenario of the aforementioned embodiments, the second preset background may be a background in a surgical scenario.

In some embodiments, post-processing may also be performed on the second image Img2 to make the second image Img2 closer to a real captured surgical scene. The post-processing may include, but is not limited to, at least one of blurring processing, sharpening processing, noise reduction processing, and enhancement processing.

In operation S16, label information of the target object Obj in the second image Img2 can be generated based on corrected pose information indicative of the corrected pose Pose1. The label information may include the corrected pose information indicative of the corrected pose Pose1. In a case that the target object Obj is the surgical instrument X, the label information may also include the type and model information of the surgical instrument X. When the second image Img2 is obtained by processing the first image Img1 through methods such as grayscale processing, background replacement, etc., the pose of the target object Obj in the second image Img2 is the same as that in the first image Img1. Therefore, the corrected pose information indicative of the corrected pose Pose1 can be directly determined as one of the label information of the target object Obj in the second image Img2. In cases where orientation transformation or scale transformation is performed on the target object Obj of the first image Img1, the pose of the target object Obj in the second image Img2 is different from that in the first image Img1. Therefore, based on a pixel mapping relationship between the second image Img2 and the first image Img1, the corrected pose Pose1 can be mapped to obtain a mapped pose, and the mapped pose can be determined as one of the label information of the target object Obj in the second image Img2.

In a case that the first image includes multiple surgical instruments X, the above process can be performed for each surgical instrument X separately, thereby obtaining label information for each surgical instrument X in the second image corresponding to the first image.

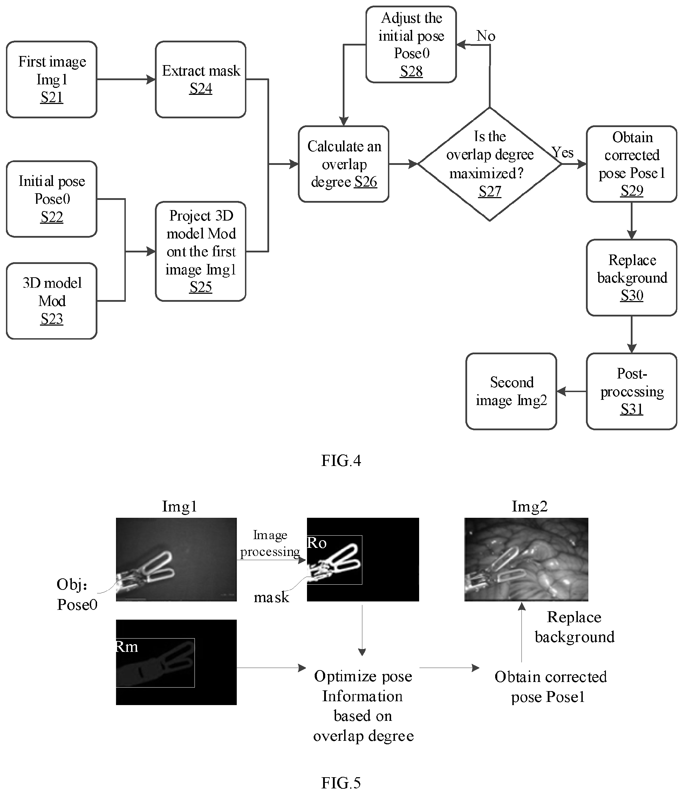

Taking the surgical scenario as an example and in conjunction with FIG. 4, an overall process of the method for generating labels for images according to embodiments of the present application is described below. The method for generating labels for images includes the following operations.

In operation S21, a first image Img1 of a surgical instrument X being against a first preset background is acquired.

In operation S22, initial pose information indicative of an initial pose Pose0 of the surgical instrument X is acquired.

In operation S23, a three-dimensional (3D) model Mod of the surgical instrument X is acquired.

In operation S24, a mask of the surgical instrument X is extracted from the first image Img1.

In operation S25, the three-dimensional model Mod is projected onto the first image Img1.

In operation S26, an overlap degree between the mask of the surgical instrument X and a projected image region of the three-dimensional model Mod on the first image Img1 is calculated.

In operation S27, whether the overlap degree is maximized is determined. If yes, execute operation S29; otherwise, execute operation S28.

In operation S28, the initial pose information indicative of the initial pose Pose0 is adjusted and the process is returned to the operation S26.

In operation S29, a pose when the overlap degree is maximized is determined as the corrected pose Pose1 to obtain corrected pose information.

In operation S30, the first preset background in the first image Img1 is replaced with a second preset background.

In operation S31, post-processing is performed on the second image Img2. The corrected pose information indicative of the corrected pose Pose1 can be regarded as label information corresponding to the post-processed second image Img2.

It should be understood that an execution order of the operations in the above method need not follow the operation numbers. For example, the operation S21, operation S22 and operation S23, operation S24 and operation S25 can be executed in parallel or in any sequential order.

FIG. 5 shows a schematic diagram of images generated in the processing flow shown in FIG. 4. First, capture the surgical instrument X being against a single-color background to obtain the first image Img1, and the three-dimensional model Mod of the target object Obj is projected onto the first image Img1 to obtain the projected pixel region Rm. The first image Img1 is segmented to obtain the mask of the surgical instrument X in the first image Img1. After performing pose optimization based on the mask and the projected pixel region Rm, the corrected pose information indicative of the corrected pose Pose1 is obtained. Then, the background in the first image Img1 is replaced with a surgical scene to obtain the second image Img2.

The above embodiment obtains the corrected pose information indicate of the corrected pose Pose1 of the target object Obj in the first image Img1 by acquiring the first image Img1 of the target object Obj, projecting the three-dimensional model Mod of the target object Obj onto the first image Img1 based on the initial pose information indicate of the initial pose Pose0 of the target object Obj to obtain the projected pixel region Rm, and then determine the corrected pose information indicative the corrected pose Pose1 based on the initial pose information indicate of the initial pose Pose0 and the overlap degree between the projected pixel region Rm and the target pixel region Ro. The label information of the target object Obj in the second image Img2 of the target object Obj generated based on the corrected pose information indicative of the corrected pose Pose1 enables acquisition of accurate label information.

In some embodiments, the second image Img2 and the label information of the target object Obj in the second image Img2 can be used to train a neural network Net. The trained neural network Net can be used to track the target object Obj. For example, during surgery, the second image Img2 obtained by the method of the aforementioned embodiments, which includes a surgical instrument as the target object and actual captured human tissues as the background, and the label information obtained by the method of the aforementioned embodiments can be used as a dataset to train the neural network Net. During surgery, the trained neural network Net can be used to track the surgical instrument X. To improve the accuracy of tracking results, a large dataset is needed to train the neural network Net. The above method can automatically and quickly generate a large dataset without relying on actual surgical operation footage. The dataset generated by the above method is rich in variety, including images of different surgical instruments in different surgical scenarios and the corrected poses of the surgical instruments in those images.

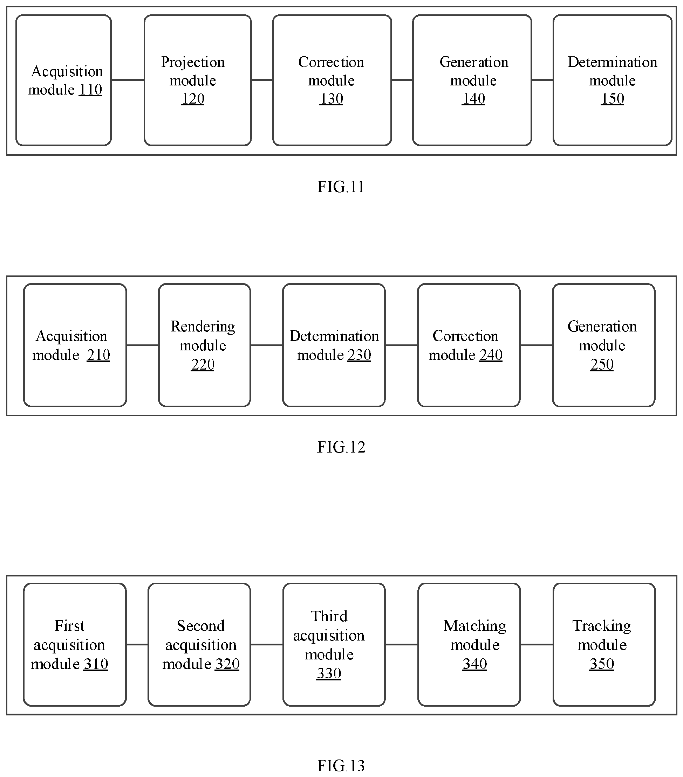

Furthermore, corresponding to the above method, embodiments of the present application also provide an apparatus for generating labels for images. Referring to FIG. 11, the apparatus includes modules as follows.

An acquisition module 110 is configured to acquire a first image Img1 of a target object Obj and initial pose information indicative of an initial pose Pose0 of the target object Obj, and is configured to acquire a three-dimensional model Mod of the target object Obj. The initial pose Pose0 is the pose of the target object Obj at the time when the first image Img1 is captured.

A projection module 120 is configured to project the three-dimensional model Mod of the target object Obj onto the first image Img1 based on the initial pose Pose0 to obtain a projected pixel region Rm.

A correction module 130 is configured to determine corrected pose information indicative of a corrected pose Pose1 based on the initial pose information indicative of the initial pose Pose0 and an overlap degree between the projected pixel region Rm and a target pixel region Ro of the target object Obj on the first image Img1.

A generation module 140 is configured to generate a second image Img2 of the target object Obj based on the first image Img1.

A determination module 150 is configured to determine label information of the target object Obj in the second image Img2 based on the corrected pose information indicative of the corrected pose Pose1.

In some embodiments, the first image is captured in a case that the target object is against a first preset background.

In some embodiments, the generation module is configured to replace the first preset background in the first image Img1 with a second preset background to obtain the second image Img2.

In some embodiments, the apparatus further includes a post-processing module configured to perform post-processing on the second image Img2. The post-processing includes at least one of blurring processing, sharpening processing, noise reduction processing, and enhancement processing.

In some embodiments, the corrected pose Pose1 is the pose of the target object Obj in a case that the overlap degree is maximized.

In some embodiments, the correction module is specifically configured to, after optimizing the initial pose information indicative of the initial pose Pose0 using a preset pose optimization algorithm, recalculate the overlap degree between the projected pixel region Rm and the target pixel region Ro, and determine the pose of the target object Obj corresponding to the overlap degree being maximum as the corrected pose Pose1.

In some embodiments, the overlap degree between the projected pixel region Rm and the target pixel region Ro is determined based on an IoU, GIoU, or dice loss between the projected pixel region Rm and the target pixel region Ro.

In some embodiments, the apparatus further includes a mask acquisition module configured to extract a mask of the target object Obj in the first image Img1, and an overlap degree determination module configured to determine the overlap degree between the mask of the target object Obj and the projected pixel region Rm.

In some embodiments, before the correction module, the apparatus further includes a smoothing processing module configured to perform smoothing processing on the mask.

In some embodiments, the target object Obj includes at least one surgical instrument X. Each surgical instrument X is held by a robotic arm 101a of a surgical robot, and the robotic arm 101a is provided with a sensor configured to detect an initial pose of the surgical instrument X held by the robotic arm. The first image Img1 is captured by an image capturing device.

In some embodiments, the three-dimensional model Mod of the surgical instrument X is associated with the type and model information of the surgical instrument X. The acquisition module is specifically configured to acquire a three-dimensional model Mod of the surgical instrument X held by the robotic arm 101a according to the type and model information of the surgical instrument X held by the robotic arm 101a.

In some embodiments, the apparatus further includes a type and model determination module configured to determine the type and model information of the surgical instrument X held by each robotic arm 101a based on an operation log of the surgical robot, or to determine the type and model information of the surgical instrument X held by each robotic arm 101a based on user input.

In some embodiments, the label information includes the corrected pose Pose1, and the type and model information of the surgical instrument X.

In some embodiments, the second image Img2 and the label information of the target object Obj in the second image Img2 are used to train a neural network. The neural network is used to track the target object Obj.

In some embodiments, functions of the apparatus provided by embodiments of the present application or modules included therein may be used to execute the methods described in the aforementioned method embodiments. For specific implementations, reference may be made to the descriptions in the aforementioned method embodiments. For brevity, details are not repeated here.

### Second embodiment.

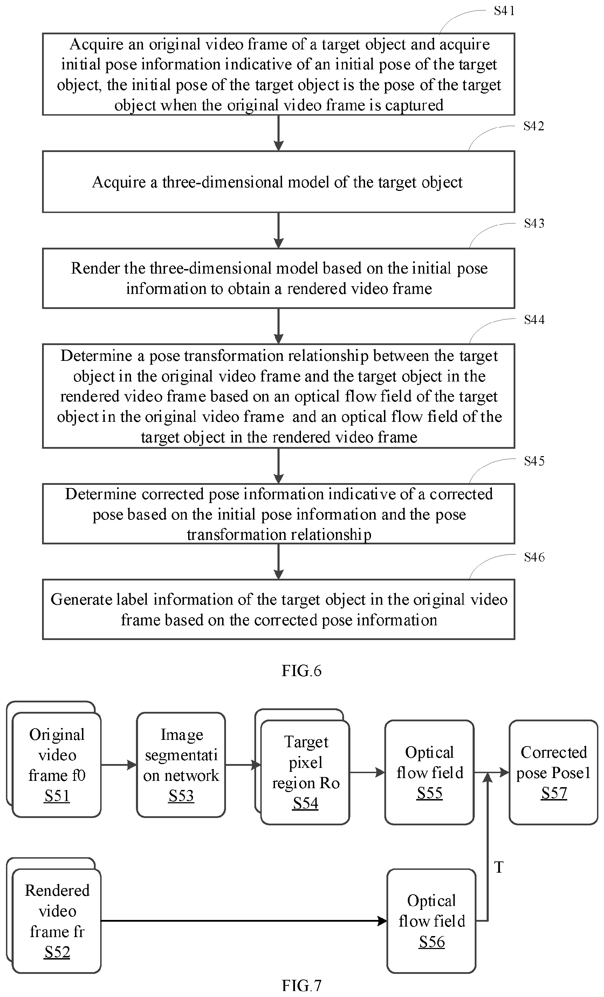

Referring to FIG. 6, embodiments of the present application further provide a method for generating labels for images. The method includes operations as follows.

In operation S41, an original video frame f0 of a target object Obj and initial pose information indicative of an initial pose Pose0 of the target object Obj are acquired. The initial pose Pose0 of the target object Obj is the pose of the target object Obj when the original video frame f0 is captured.

In operation S42, a three-dimensional model Mod of the target object Obj is acquired.

In operation S43, the three-dimensional model Mod is rendered based on the initial pose Pose0 to obtain a rendered video frame fr.

In operation S44, a pose transformation relationship T between the target object Obj in the original video frame f0 and the target object Obj in the rendered video frame fr is determined based on an optical flow field of the target object Obj in the original video frame f0 and an optical flow field of the target object Obj in the rendered video frame fr.

In operation S45, corrected pose information indicative of a corrected pose Pose1 is determined based on the initial pose information indicative of the pose Pose0 and the pose transformation relationship T.

In operation S46, label information of the target object Obj in the original video frame f0 is generated based on the corrected pose information indicative of the corrected pose Pose1.

In operation S41, the original video frame f0 may include one or more video frames from a video. The original video frame f0 may be a video frame captured in a specified scenario. Generally, the specified scenario refers to an actual application scenario of the target object Obj, for example, a surgical scenario. In the original video frame f0, besides including the target object Obj, it may also include a background of the specified scenario. For example, when the specified scenario is a surgical scenario, the background of the specified scenario may include tissues inside a human or animal body undergoing surgery. In some embodiments, each original video frame f0 may be the first image Img1 in the aforementioned first embodiment.

The target object Obj may be a surgical instrument X or other objects. The specific category of the target object Obj may vary depending on the actual application scenario. In embodiments in a case that the target object Obj is a surgical instrument X, the surgical instrument X may be held by a robotic arm 101a of a surgical robot. The robotic arm 101a of the surgical robot may also hold an image capturing device for capturing the original video frame f0. Alternatively, the image capturing device for capturing the original video frame f0 may also be mounted on a stand or fixed at other locations (e.g., wall, table, or patient bedside). A pose sensing device may be provided on the robotic arm 101a holding the surgical instrument X to detect an initial pose Pose0 of the surgical instrument X at the time when the first image Img1 is captured. In one embodiment, a pose sensing device may also be provided on the robotic arm 101a holding the image capturing device to detect the pose of the image capturing device. In one embodiment, the robotic arm 101a includes a plurality of links sequentially connected, with adjacent links connected via rotary joints. The pose sensing device may include encoders provided at the rotary joints for measuring relative rotation angles between adjacent links.

In operation S42, a three-dimensional model Mod of the surgical instrument X is acquired. Specific embodiments of the operation S42 can be referred to the description of the operation S12 above and are not repeated here.

In operation S43, the three-dimensional model Mod may be a three-dimensional model in a physical coordinate system. For example, it may be a standard model located at an origin of a physical coordinate system with a specified posture. In a case that the target object is the surgical instrument X, the initial pose Pose0 of the surgical instrument X may be a pose measured by encoders on the robotic arm where the surgical instrument X is located. This pose may be the pose of the surgical instrument X in a physical coordinate system such as a PSR base coordinate system or a world coordinate system. Based on the above initial pose Pose0, the three-dimensional model Mod can be rendered to obtain a rendered video frame fr including the three-dimensional model Mod. During rendering, the three-dimensional model Mod can be projected into a coordinate system of the image capturing device according to the initial pose Pose0 to obtain a projected video frame, and then the projected video frame can be rendered to obtain the rendered video frame fr. For example, based on a transformation matrix of the image capturing device, the initial pose Pose0 can be converted into a pose in the coordinate system of the image capturing device, and then the three-dimensional model Mod can be projected into the coordinate system of the image capturing device based on the converted pose to obtain a projected video frame. The transformation matrix characterizes a conversion relationship between the coordinate system of the image capturing device and the physical coordinate system. After obtaining the projected video frame, rendering can be performed on the target object Obj in the projected video frame based on a pre-generated color map and a pre-generated texture map.

Assuming the number of original video frames f0 is N (N is a positive integer), the three-dimensional model Mod can be rendered based on the initial pose Pose0 of the target object Obj at the time when each original video frame f0 is captured, separately, to obtain N rendered video frames fr. Each rendered video frame fr corresponds to one original video frame f0.

In operation S44, an optical flow field (Optical Flow) is used to describe motion information in an image sequence. The optical flow field can be understood as a displacement amount of each pixel in the image over time. Based on the optical flow field of the target object Obj in the original video frame f0 and the optical flow field of the target object Obj in the rendered video frame fr, motion and change situations between the target object Obj in the original video frame f0 and the target object Obj in the rendered video frame fr can be analyzed to determine a pose difference of the target object Obj between the two video frames. This pose difference can be characterized by the pose transformation relationship T (which can be a transformation matrix). For example, a RANSAC (random sample consensus) algorithm or a two-dimensional template matching algorithm can be used to estimate the above-mentioned pose transformation relationship T.

In some embodiments, image segmentation may also be performed on the original video frame f0 to obtain a target pixel region Ro of the target object Obj on the original video frame f0. The operation of performing image segmentation on the original video frame f0 may be implemented by a pre-trained image segmentation network, or image segmentation may be performed based on information manually annotated by a user. By performing image segmentation, a background region in the original video frame can be removed, retaining only the target pixel region Ro of the target object Obj on the original video frame f0, thereby reducing the influence of background region on subsequent operations. Similarly, a target pixel region Ro of the target object Obj on a subsequent video frame of the original video frame f0 can also be acquired. The subsequent video frame of the original video frame f0 may be a video frame that is the h-th (h is a positive integer) frame after the original video frame f0. For example, assuming the original video frame f0 is the first frame in a video, the subsequent video frame of the original video frame f0 may be the second frame or a later frame in that video. Similar to the processing of the original video frame f0, image segmentation may also be performed on the subsequent video frame of the original video frame f0 to obtain the target pixel region Ro of the target object Obj on the subsequent video frame of the original video frame f0. Then, the optical flow field of the target object Obj in the original video frame f0 can be determined based on the target pixel region Ro of the target object Obj on the original video frame f0 and the target pixel region Ro of the target object Obj on the subsequent video frame of the original video frame f0.

In other embodiments, image segmentation may not be performed, and the optical flow field of the target object Obj in the original video frame f0 and the optical flow field of the target object Obj in the rendered video frame fr may be obtained directly.

In operation S45, the initial pose Pose0 is transformed based on the pose transformation relationship T acquired in operation S44 to obtain the corrected pose Pose1.

Operation S46 may be referred to operation S16 in first embodiment and is not repeated here.

Embodiments of the present disclosure obtain the corrected pose Pose1 by acquiring the optical flow field of the target object Obj in the original video frame f0, and after rendering the three-dimensional model Mod of the target object Obj based on the initial pose Pose0 of the target object Obj when the original video frame f0 is captured to obtain the rendered video frame fr, acquiring the optical flow field of the target object Obj in the rendered video frame fr, and then determining the pose transformation relationship T between the target object Obj in the original video frame f0 and the target object Obj in the rendered video frame fr based on the two optical flow fields. This pose transformation relationship T can reflect the difference between motion information of the target object Obj in the original video frame f0 and motion information of the target object Obj in the rendered video frame fr. Therefore, correcting the initial pose Pose0 based on the pose transformation relationship T yields a corrected pose Pose1, thereby making the label information generated based on the corrected pose Pose1 more accurate.

In some embodiments, the label information may include the corrected pose Pose1. In a case that the target object Obj is the surgical instrument X, the label information may also include the type and model information of the surgical instrument X.

Taking the surgical scenario as an example and in conjunction with FIG. 7, an overall process of the method for generating labels for images according to embodiments of the present application is described below. The method for generating labels for images includes the following operations.

In operation S51, an original video frame f0 is acquired.

In operation S52, a rendered video frame fr is acquired.

In operation S53, the original video frame f0 is input into an image segmentation network for image segmentation.

In operation S54, a foreground region (i.e., a target pixel region Ro of the target object Obj) in the original video frame f0 is acquired through the image segmentation network.

In operation S55, an optical flow field of the target object Obj in the original video frame f0 is calculated.

In operation S56, an optical flow field of the target object Obj in the rendered video frame fr is calculated.

In operation S57, corrected pose information indicative of a corrected pose Pose1 is determined based on the initial pose information indicative of the initial pose Pose0 of the target object Obj in the original video frame f0 and the optical flow field of the target object Obj in the original video frame f0 and the optical flow field of the target object Obj in the rendered video frame fr, and the corrected pose information indicative of the corrected pose Pose1 is regarded as label information corresponding to the original video frame f0.

It should be understood that an execution order of the operations in the above method need not follow the operation numbers. For example, the operation S51 and operation S52, operation S55 and operation S56 can be executed in parallel or in any sequential order.

In some embodiments, the original video frame f0 and the label information of the target object Obj in the original video frame f0 can be used to train a neural network Net. This neural network Net can be used to track the target object Obj. For example, in a surgical scenario, video frames from actual surgical operation videos and label information obtained by the method of the aforementioned embodiments can be used as a dataset to train the neural network Net. During a surgical procedure, the trained neural network Net can be used to track surgical instruments X. To improve the accuracy of tracking results, a large dataset is needed to train the neural network Net. The above method can automatically and quickly generate a large dataset based on recorded real surgical operation videos. The generated dataset better reflects real surgical operation scenarios and can improve the quality of neural network Net training.

Furthermore, corresponding to the above method, embodiments of the present application also provide an apparatus for generating labels for images. Referring to FIG. 12, the apparatus as follows.

An acquisition module 210 is configured to acquire an original video frame f0 of a target object Obj and initial pose information indicative of an initial pose Pose0 of the target object Obj, and is configured to acquire a three-dimensional model Mod of the target object Obj. The initial pose Pose0 of the target object Obj is the pose of the target object Obj at the time when the original video frame f0 is captured.

A rendering module 220 is configured to render the three-dimensional model Mod based on the initial pose information indicative of the initial pose Pose0 to obtain a rendered video frame fr.

A determination module 230 is configured to determine a pose transformation relationship T between the target object Obj in the original video frame f0 and the target object Obj in the rendered video frame fr based on an optical flow field of the target object Obj in the original video frame f0 and an optical flow field of the target object Obj in the rendered video frame fr.

A correction module 240 is configured to determine corrected pose information indicative of a corrected pose Pose1 based on the initial pose information indicative of the initial pose Pose0 and the pose transformation relationship T.

A generation module 250 is configured to generate label information of the target object Obj in the original video frame f0 based on the corrected pose information indicative of the corrected pose Pose1.

In some embodiments, the apparatus further includes an image segmentation module configured to perform image segmentation on the original video frame f0 to obtain a target pixel region Ro of the target object Obj on the original video frame f0, and an optical flow field determination module configured to determine an optical flow field of the target object Obj in the original video frame f0 based on the target pixel region Ro of the target object Obj on the original video frame f0 and a target pixel region Ro of the target object Obj on a subsequent video frame of the original video frame f0.

In some embodiments, the rendering module is configured to project the three-dimensional model Mod into a coordinate system of an image capturing device according to the initial pose information indicative of the initial pose Pose0 to obtain a projected video frame, and render the projected video frame to obtain the rendered video frame.

In some embodiments, the target object Obj includes at least one surgical instrument X. Each surgical instrument X is held by a robotic arm 101a of a surgical robot, and the robotic arm 101a is provided with a sensor configured to detect the initial pose Pose0 of the surgical instrument X held by the robotic arm. The original video frame f0 is captured by an image capturing device.

In some embodiments, the three-dimensional model Mod of the surgical instrument X corresponds to the type and model information of the surgical instrument X. The acquisition module is configured to acquire a three-dimensional model Mod of the surgical instrument X held by the robotic arm 101a according to the type and model information of the surgical instrument X held by the robotic arm 101a.

In some embodiments, the apparatus further includes a type and model determination module configured to determine the type and model information of the surgical instrument X held by each robotic arm 101a based on an operation log of the surgical robot, or to determine the type and model information of the surgical instrument X held by each robotic arm 101a based on user input.

In some embodiments, the label information includes the corrected pose Pose1, and the type and model information of the surgical instrument X.

In some embodiments, the original video frame f0 and the label information of the target object Obj in the original video frame f0 are used to train a neural network. The neural network is used to track the target object Obj.

In some embodiments, functions of the apparatus provided by embodiments of the present application or modules included therein may be used to execute the methods described in the aforementioned method embodiments. For specific implementations, reference may be made to the descriptions in the aforementioned method embodiments. For brevity, details are not repeated here.

Embodiments of the present application can generate a large number of accurate pose information based on noisy initial pose information. In related technologies, obtaining accurate pose information requires precise control of robotic arms or manual annotation, which is costly. Using the first embodiment or the second embodiment of the present disclosure can effectively improve pose acquisition accuracy while reducing costs.

Neural network model and tracking of the target object are introduced below.

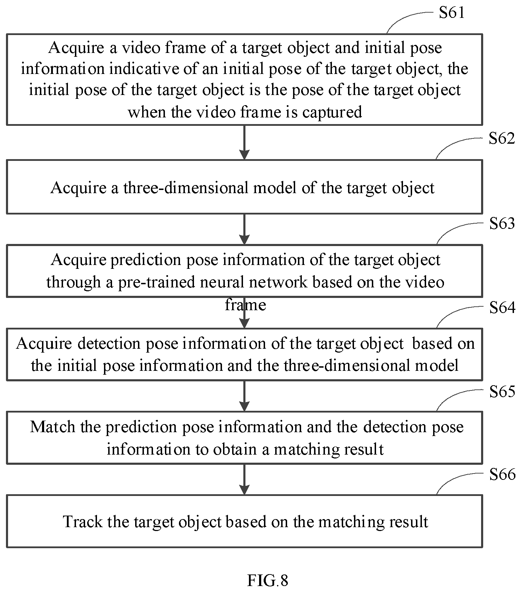

Referring to FIG. 8, embodiments of the present disclosure also provide a method for tracking a target object Obj. The method includes operations as follows.

In operation S61, a video frame f of a target object Obj and initial pose information indicative of an initial pose Pose0 of the target object Obj are acquired. The initial pose Pose0 of the target object Obj is the pose of the target object Obj at the time when the video frame f is captured.

In operation S62, a three-dimensional model Mod of the target object Obj is acquired.

In operation S63, prediction pose information Poseₚᵣₑ of the target object Obj is acquired through a pre-trained neural network Net based on the video frame f.

In operation S64, detection pose information Pose_{det} of the target object Obj is acquired based on the initial pose information Pose0 and the three-dimensional model Mod.

In operation S65, the prediction pose information Poseₚᵣₑ and the detection pose information Pose_{det} are matched to obtain a matching result.

In operation S66, the target object Obj is tracked based on the matching result.

In operation S61, the target object Obj may be a surgical instrument X or other objects. The video frame f of the surgical instrument X can be from a real video captured by an image capturing device. The surgical instrument X and the image capturing device may both be held by robotic arms 101a of a surgical robot. The image capturing device may also be mounted on a stand or fixed at other locations (e.g., wall, table, or patient bedside). A pose sensing device may be provided on the robotic arm 101a holding the surgical instrument X to detect an initial pose Pose0 of the surgical instrument X held by that robotic arm 101a at the time when the video frame f is captured to obtain initial pose information. In an example, the robotic arm 101a includes a plurality of links sequentially connected, with adjacent links connected via rotary joints. The pose sensing device may include encoders provided at the rotary joints for detecting relative rotation angles between adjacent links.

In operation S62, a three-dimensional model Mod of the target object Obj can be acquired. Specific embodiments of the operation S62 can be referred to the description of operation S12 above and are not repeated here.

In operation S63, prediction pose information Poseₚᵣₑ of the target object Obj in the video frame f can be acquired through the pre-trained neural network Net. This neural network Net can be trained based on sample images and label information of the sample images. The sample images may be the second images Img2 in first embodiment, and correspondingly, the label information of the sample images can be acquired based on the method in first embodiment. Alternatively, the sample images may be the original video frames f0 in second embodiment, and correspondingly, the label information of the sample images can be acquired based on the method in the second embodiment.

The prediction pose information Poseₚᵣₑ acquired by the neural network Net may include a prediction pixel region, prediction keypoint information, and prediction orientation information of the target object Obj. The prediction pixel region is a pixel region of the target object Obj on the video frame. The prediction keypoint information may include position information of one or more keypoints of the target object Obj in the video frame. The prediction orientation information indicates a posture of the target object Obj in the video frame and may include a yaw angle, a roll angle, and/or a pitch angle of the target object Obj.

In some embodiments, a bounding box of the target object Obj in the video frame f can be acquired, and the prediction pose information Poseₚᵣₑ of the target object Obj in the video frame f can be acquired based on the bounding box of the target object Obj in the video frame f.

In some embodiments, the bounding box of the target object Obj in the video frame f can be acquired based on the video frame f and a tracking result of the target object Obj in a video frame fₚᵣᵢₒᵣ prior to the video frame f. The prior video frame fₚᵣᵢₒᵣ may include at least one video frame prior to the video frame f in the video to which the video frame f belongs. Assuming the video frame f is the m-th frame (m is a positive integer) in a video, the prior video frame fₚᵣᵢₒᵣ may include at least one of: the (m-1)-th video frame, the (m-2)-th video frame, the (m-3)-th video frame, etc., in the video. The tracking result of the target object Obj in the prior video frame fₚᵣᵢₒᵣ may include a detection result of the bounding box of the target object Obj in the prior video frame fₚᵣᵢₒᵣ. Since a position of the target object Obj is constrained by the physical world and does not change abruptly, acquiring the bounding box of the target object Obj in the video frame f based on the tracking result of the target object Obj in the prior video frame fₚᵣᵢₒᵣ can effectively improve the accuracy of bounding box acquisition.

After acquiring the bounding box of the target object Obj, pooling processing can be performed on features within the bounding box of the target object Obj in the video frame f to obtain the prediction pose information Poseₚᵣₑ of the target object Obj. The pooling processing may employ methods such as max pooling or average pooling. Performing pooling processing helps in locating the target object during pose estimation. After locating the target object, the bounding box of the target object can be restored to its original size, and then calculation of the prediction pose information Poseₚᵣₑ can be performed.

In some embodiments, the aforementioned neural network Net may include a plurality of feature extraction layers l_{f} configured to perform feature extraction on the video frame f. Features output from at least one first feature extraction layer among the plurality of feature extraction layers l_{f} are used to acquire the bounding box of the target object Obj in the video frame. Features output from at least one second feature extraction layer among the plurality of feature extraction layers l_{f} are used to acquire the prediction pose information Poseₚᵣₑ of the target object Obj in the video frame. Each second feature extraction layer is prior to each first feature extraction layer.

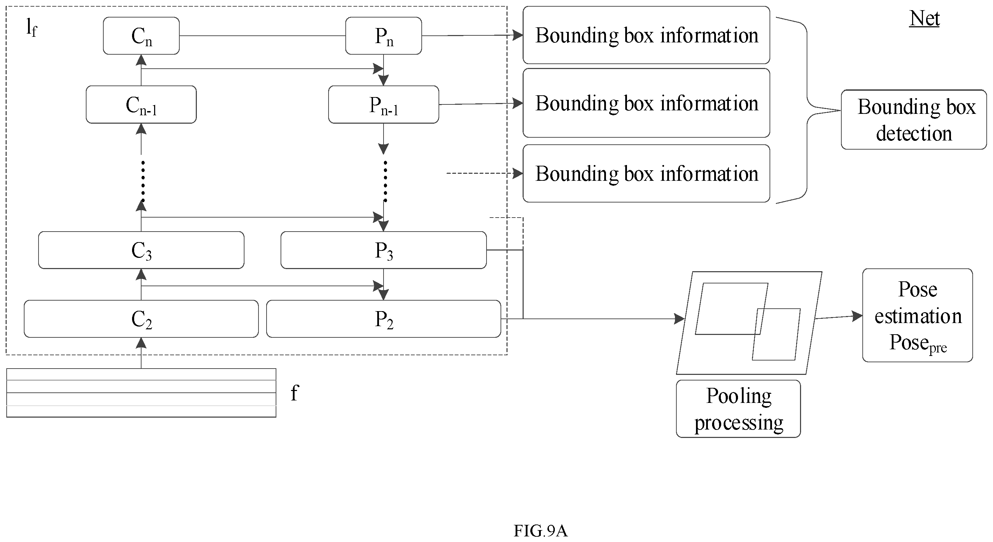

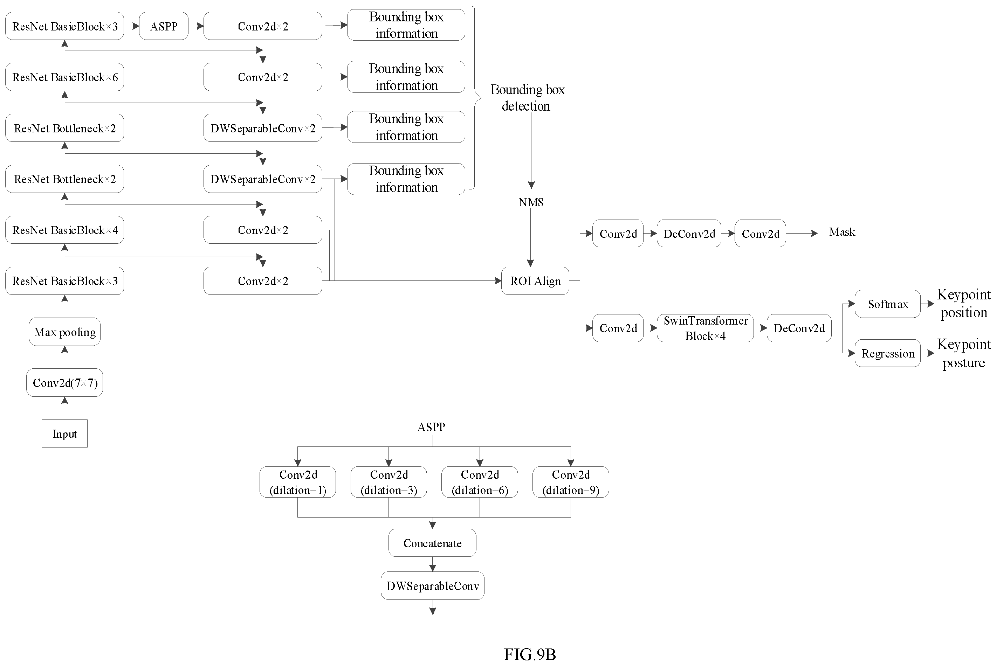

FIG. 9A and FIG. 9B show an architecture of the neural network Net according to embodiments of the present disclosure. Assuming the neural network Net includes a total of n-1 (n is a positive integer, and n > 1) feature extraction layers l_{f}, features output from the first to the k-th (k is a positive integer, and k < n-1) feature extraction layers l_{f} are used to acquire the prediction pose information Poseₚᵣₑ of the target object Obj in the video frame. Features output from the (k+1)-th to the (n-1)-th feature extraction layers l_{f} are used to acquire the bounding box of the target object Obj in the video frame. Embodiments of the present disclosure use a two-stage model as the neural network Net for bounding box detection and pose estimation. In a first stage, high-level features are used to detect the bounding box from the video frame, which can acquire more feature information, thereby improving the accuracy of bounding box detection. In a second stage, low-level features are used for pose estimation on features within the bounding box box. Low-level features are typically associated with geometric structures in the video frame. By using low-level features, the neural network Net can acquire edge, corner, and other geometric structure information from the video frame, improving the accuracy of pose estimation. Furthermore, low-level features generally have better stability and are less susceptible to factors such as lighting changes and noise, improving the performance stability of pose estimation. Moreover, bounding box information can be detection based on features output from each of the (k+1)-th to the (n-1)-th feature extraction layers l_{f}. The detection bounding box information includes geometric information (width and height) of the bounding box and a confidence score corresponding to the bounding box. By performing non-maximum suppression (NMS) on the detection bounding boxes, the bounding box with the highest confidence score can be determined as the bounding box of the target object Obj, and other bounding boxes can be filtered out. This can effectively improve the detection accuracy of the bounding box.

In some embodiments, as shown in FIG. 9A and FIG. 9B, each feature extraction layer l_{f} includes an encoder and a decoder. An output end of an encoder Cᵢ of an i-th feature extraction layer l_{f} is connected to an input end of an encoder Cᵢ₊₁ of an (i+1)-th feature extraction layer lf and an input end of a decoder Pᵢ of the i-th feature extraction layer l_{f}. An input end of the decoder Pᵢ of the i-th feature extraction layer l_{f} is connected to an output end of a decoder Pᵢ₊₁ of the (i+1)-th feature extraction layer l_{f}. Where i is a positive integer. Each encoder is configured to perform down-sampling processing on features input thereto. Each decoder is configured to perform up-sampling processing on features input thereto. Each feature encoder and feature decoder may include a convolutional neural network Net, multiple transformer layers, or multiple pooling layers. Each encoder performs down-sampling on features output from an encoder of a previous feature extraction layer l_{f} to reduce feature dimensions. Each decoder performs up-sampling on features output from a decoder of a previous feature extraction layer l_{f} and an encoder of a corresponding feature extraction layer l_{f} to restore feature dimensions, thereby obtaining high-resolution pose prediction results.

Referring to FIG. 9B, the encoders include an encoder formed by connecting several bottleneck structures of a ResNet network, and an encoder formed by connecting several BasicBlock structures of the ResNet network. Each decoder may be formed by connecting several convolutional layers. For example, the encoder of the first feature extraction layer in the figure is formed by connecting three BasicBlock structures. The encoder of the second feature extraction layer is formed by connecting four BasicBlock structures. The encoders of the third and fourth feature extraction layers are both formed by connecting two bottleneck structures. The BasicBlock structure and bottleneck structure are shown as ResNet BasicBlock and ResNet Bottleneck in the figure, respectively. For the "×x" in each rectangular box, the number "x" following the multiplication sign indicates a quantity of corresponding structures. For example, in the encoder of the first feature extraction layer, ResNet BasicBlock×3 indicates that the encoder is formed by connecting three BasicBlock structures.

If an encoder of a feature extraction layer is formed by connecting bottleneck structures, then a decoder of that feature extraction layer is formed by connecting several depthwise separable convolution layers. In the embodiment shown in FIG. 9B, the decoders of the third and fourth feature extraction layers are both formed by connecting two depthwise separable convolution layers, shown as DWSeparableConv in the figure. Conv2d in the figure represents two-dimensional convolution. By increasing encoders formed by connecting bottleneck structures and decoders formed by connecting depthwise separable convolution layers, the number of feature extraction layers can be increased, thereby extracting more features.

An atrous spatial pyramid pooling (ASPP) module may also be included between the encoder of the last feature extraction layer and the decoder of the last feature extraction layer. The ASPP module first uses multiple parallel convolution modules to perform atrous convolution processing on features output from a last encoder respectively. Different convolution modules use different dilation rate parameters (dilation) to obtain different receptive fields. The ASPP module shown in the figure uses four parallel convolution modules with dilation rate parameters of 1, 3, 6, and 9, respectively.

Then, features output from each convolution module are concatenated (concat), and depthwise separable convolution is performed on the concatenated features. The features obtained through depthwise separable convolution can be output to the decoder of the last feature extraction layer. Using the ASPP module can increase a receptive field, thereby extracting more features.

Information of the bounding box obtained after NMS processing can be output to a pooling processing layer, enabling this pooling processing layer to perform pooling processing on features output from the first to the k-th (k is a positive integer, and k < n-1) feature extraction layers l_{f} based on the bounding box information. The pooling processing layer may use ROI align to perform pooling processing on the acquired features. Features after pooling processing are used on one hand to extract a mask of the target object, and on the other hand to acquire the prediction pose information Poseₚᵣₑ of the target object (including prediction keypoint positions and prediction keypoint posture of the target object). Conv2d and DeConv2d in the figure represent two-dimensional convolution and two-dimensional deconvolution processing, respectively. Softmax is normalization processing. Regression represents regression processing. When determining keypoint positions and posture, features output from each feature extraction layer can be processed through a swin transformer to effectively extract global features and improve the accuracy of keypoint detection.

It should be understood that the structure shown in the figure is merely an exemplary structure of the neural network. Besides the above structure, other structures of neural networks may also be used in embodiments of the present disclosure, which are not repeated here.

In operation S64, corresponding to the prediction pose information, the detection pose information may include a detection pixel region, detection keypoint information, and detection orientation information of the target object Obj.

In some embodiments, the three-dimensional model Mod can be projected into a coordinate system of the image capturing device according to the initial pose information indicative of the initial pose Pose0, and detection keypoint information and detection orientation information of the target object Obj in the coordinate system of the image capturing device can be acquired. Additionally, the three-dimensional model Mod can be projected onto a two-dimensional image plane corresponding to the video frame according to the initial pose information indicative of the initial pose Pose0, and a detection pixel region of the target object Obj within the two-dimensional image plane can be acquired.

The initial pose Pose0 may characterize a relative pose between the three-dimensional model Mod and the image capturing device. Based on the initial pose information indicative of the initial pose Pose0, three-dimensional keypoints on the three-dimensional model Mod can be projected into the coordinate system of the image capturing device to obtain the detection keypoint information and detection orientation information. Furthermore, based on the initial pose information indicative of the initial pose Pose0, the three-dimensional model Mod can be projected onto the two-dimensional image plane corresponding to the video frame, and the projected image on the two-dimensional image plane can be detection through the aforementioned neural network Net to obtain the detection pixel region.

In operation S65, the prediction pixel region and the detection pixel region can be matched to obtain a pixel region matching result, the prediction keypoint information and the detection keypoint information can be matched to obtain a keypoint matching result, and the prediction orientation information and the detection orientation information can be matched to obtain an orientation information matching result. For example, bipartite matching can be used to implement the matching of the aforementioned items of information.

In operation S66, a first confidence score that the prediction pixel region matches the detection pixel region can be determined based on the pixel region matching result. A second confidence score that the prediction keypoint information matches the detection keypoint information can be determined based on the keypoint matching result. A third confidence score that the prediction orientation information matches the detection orientation information can be determined based on the orientation information matching result. Then, the target object Obj can be tracked based on the first confidence score, the second confidence score, and the third confidence score.

For example, weighted averaging can be performed on the first confidence score, the second confidence score, and the third confidence score to obtain a weighted average confidence score. If the weighted average confidence score is greater than a preset confidence threshold, the matching is determined to be successful, and the initial pose of the target object Obj is corrected based on the prediction pose information, and the correction result is saved. If the weighted average confidence score is less than or equal to the preset confidence threshold, the matching is determined to be unsuccessful.

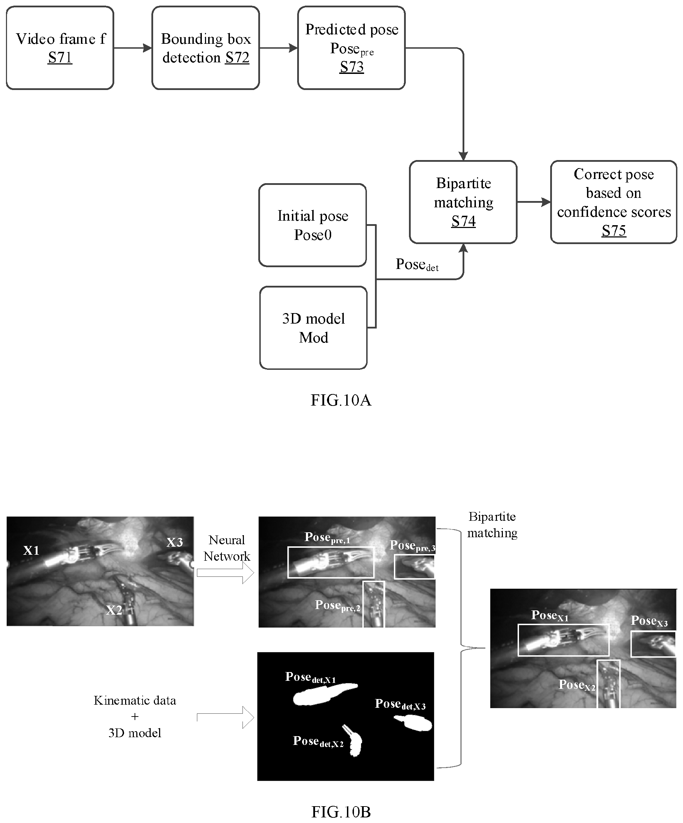

Taking a surgical scenario as an example and in conjunction with FIG. 10A, an overall process of the method for tracking a target object Obj in an image according to embodiments of the present application is described below. The method for tracking a target object Obj includes the following operations.

In operation S71, a video frame f of a surgical instrument X is acquired.

In operation S72, bounding box detection is performed on the video frame f to obtain a bounding box of the surgical instrument X in the video frame f.

In operation S73, pose prediction is performed on the surgical instrument X in the video frame f based on the bounding box obtained in operation S72 to obtain prediction pose information Poseₚᵣₑ.

In operation S74, detection pose information Pose_{det} is acquired based on a three-dimensional model Mod of the surgical instrument X and initial pose information indicative of an initial pose Pose0 of the surgical instrument X, and bipartite matching is performed on the prediction pose information Poseₚᵣₑ and the detection pose information Pose_{det}.

In operation S75, corrected pose information indicative of a corrected pose Pose1 is determined based on the initial pose information indicative of the initial pose Pose0, the matching result, and confidence scores.

Embodiments of the present disclosure effectively improve tracking accuracy by acquiring prediction pose information of a target object through a neural network, acquiring detection pose information of the target object based on initial pose information and a three-dimensional model of the target object, matching the prediction pose information and the detection pose information, and tracking the target object based on the matching result.

Using the tracking method of embodiments of the present application, a single target object Obj can be tracked, and multiple target objects Obj can also be tracked.

A multi-target tracking process is illustrated below with the example shown in FIG. 10B. In this example, three surgical instruments X (shown as X1, X2, and X3 in the figure) need to be tracked. Therefore, after inputting the video frame f into the neural network, three sets of prediction pose information Poseₚᵣₑ (shown as Pose_{pre,1}, Pose_{pre,2}, and Pose_{pre,3} in the figure) are obtained. Corresponding detection pose information of the surgical instruments X1, X2, and X3 can be obtained based on kinematic data (including initial poses) and three-dimensional models of the surgical instruments X1, X2, and X3, denoted as Pose_{det,X1}, Pose_{det,X2}, and Pose_{det,X3}, respectively. Bipartite matching is performed for each of the three sets of prediction pose information {Pose_{pre,1}, Pose_{pre,2}, Pose_{pre,3}} with each of the three sets of detection pose information {Pose_{det,X1}, Pose_{det,X2}, Pose_{det,X3}}, and a matching confidence score is calculated. The matching confidence score is compared with a preset confidence threshold to determine a matching result. For example, in response to the matching confidence score being greater than the preset confidence threshold, the matching is determined to be successful, and the initial pose is corrected according to the prediction position information. In one example, in response to prediction pose information Pose_{pre,1}, and detection pose information Pose_{det,X1} are successfully matched, prediction pose information Pose_{pre,1} is determined as the prediction pose information corresponding to surgical instrument X1, and prediction pose information Pose_{pre,1} is used to correct the initial pose of surgical instrument X1. For example, a pose offset between prediction pose information Pose_{pre,1} and the initial pose can be saved. The processing for the other two surgical instruments X2 and X3 is similar and will not be repeated here. Finally, tracking results for surgical instruments X1, X2, and X3 can be obtained, as shown by Pose_{X1}, Pose_{X2}, and Pose_{X3} in the figure.

Furthermore, corresponding to the above method, embodiments of the present application also provide an apparatus for tracking a target object Obj. Referring to FIG. 13, the apparatus includes modules as follows.

A first acquisition module 310 is configured to acquire a video frame f of a target object Obj and initial pose information indicative of an initial pose Pose0 of the target object Obj, and is configured to acquire a three-dimensional model Mod of the target object Obj. The initial pose Pose0 of the target object Obj is the pose of the target object Obj at the time when the video frame f is captured.

A second acquisition module 320 is configured to acquire prediction pose information _{Poseₚᵣₑ} of the target object Obj through a pre-trained neural network Net based on the video frame f.

A third acquisition module 330 is configured to acquire detection pose information Pose_{det} of the target object Obj based on the initial pose information indicative of the initial pose Pose0 and the three-dimensional model Mod.

A matching module 340is configured to match the prediction pose information Poseₚᵣₑ and the detection pose information Pose_{det} to obtain a matching result.

A tracking module 350 is configured to track the target object Obj based on the matching result.

In some embodiments, the prediction pose information Poseₚᵣₑ includes a prediction pixel region, prediction keypoint information, and prediction orientation information of the target object. The detection pose information Pose_{det} includes a detection pixel region, detection keypoint information, and detection orientation information of the target object. The matching module is specifically configured to match the prediction pixel region and the detection pixel region, match the prediction keypoint information and the detection keypoint information, and match the prediction orientation information and the detection orientation information.

In some embodiments, the matching result includes a pixel region matching result, a keypoint matching result, and an orientation information matching result. The tracking module is specifically configured to determine a first confidence score that the prediction pixel region matches the detection pixel region based on the pixel region matching result, determine a second confidence score that the prediction keypoint information matches the detection keypoint information based on the keypoint matching result, determine a third confidence score that the prediction orientation information matches the detection orientation information based on the orientation information matching result, and track the target object based on the first confidence score, the second confidence score, and the third confidence score.

In some embodiments, the third acquisition module is configured to project the three-dimensional model Mod into a coordinate system of an image capturing device according to the initial pose information indicative of the initial pose Pose0, and acquire detection keypoint information and detection orientation information of the target object Obj in the coordinate system of the image capturing device, where the image capturing device is configured to capture the video frame f, and project the three-dimensional model Mod onto a two-dimensional image plane corresponding to the video frame f according to the initial pose information indicative of the initial pose Pose0, and acquire a detection pixel region of the target object Obj within the two-dimensional image plane.

In some embodiments, the second acquisition module is configured to acquire a bounding box of the target object Obj in the video frame f, and acquire the prediction pose information Poseₚᵣₑ of the target object Obj in the video frame f based on the bounding box of the target object Obj in the video frame f.

In some embodiments, the neural network Net includes a plurality of feature extraction layers l_{f} configured to perform feature extraction on the video frame f. Features output from at least one first feature extraction layer among the plurality of feature extraction layers l_{f} are used to acquire the bounding box of the target object in the video frame f. Features output from at least one second feature extraction layer among the plurality of feature extraction layers l_{f} are used to acquire the prediction pose information of the target object Obj in the video frame f. Each second feature extraction layer is prior to each first feature extraction layer.

In some embodiments, each of the feature extraction layers includes an encoder and a decoder. An output end of an encoder of an i-th feature extraction layer is connected to an input end of an encoder of an (i+1)-th feature extraction layer and an input end of a decoder of the i-th feature extraction layer. An input end of the decoder of the i-th feature extraction layer is connected to an output end of a decoder of the (i+1)-th feature extraction layer. Where i is a positive integer. Each encoder is configured to perform down-sampling processing on features input thereto. Each decoder is configured to perform up-sampling processing on features input thereto.

In some embodiments, the neural network Net acquires the bounding box of the target object Obj in the video frame f by acquiring the bounding box of the target object Obj in the video frame f based on the video frame f and a tracking result of the target object Obj in a prior video frame of the video frame f.

In some embodiments, the neural network Net acquires the prediction pose information Poseₚᵣₑ of the target object Obj in the video frame f by performing pooling processing on features within the bounding box of the target object Obj in the video frame f to obtain the prediction pose information Poseₚᵣₑ of the target object Obj.

In some embodiments, the target object Obj includes at least one surgical instrument X. Each surgical instrument X is held by a robotic arm 101a of a surgical robot, and the robotic arm 101a is provided with a sensor configured to detect an initial pose Pose0 of the surgical instrument X. The video frame f is captured by an image capturing device.

In some embodiments, the three-dimensional model Mod of the surgical instrument X corresponds to the type and model information of the surgical instrument X. The first acquisition module is specifically configured to acquire a three-dimensional model Mod of the surgical instrument X held by the robotic arm 101a according to the type and model information of the surgical instrument X held by the robotic arm 101a.

In some embodiments, the apparatus further includes a type and model acquisition module configured to determine the type and model information of the surgical instrument X held by each robotic arm 101a based on an operation log of the surgical robot, or to determine the type and model information of the surgical instrument X held by each robotic arm 101a based on user input.

In some embodiments, functions of the apparatus provided by embodiments of the present application or modules included therein may be used to execute the methods described in the aforementioned method embodiments. For specific implementations, reference may be made to the descriptions in the aforementioned method embodiments. For brevity, details are not repeated here.

Embodiments of the present application also provide a computing device, which at least includes a memory, a processor, and a computer program stored on the memory and executable on the processor. When the processor executes the program, the method according to any embodiment of the present application is implemented.

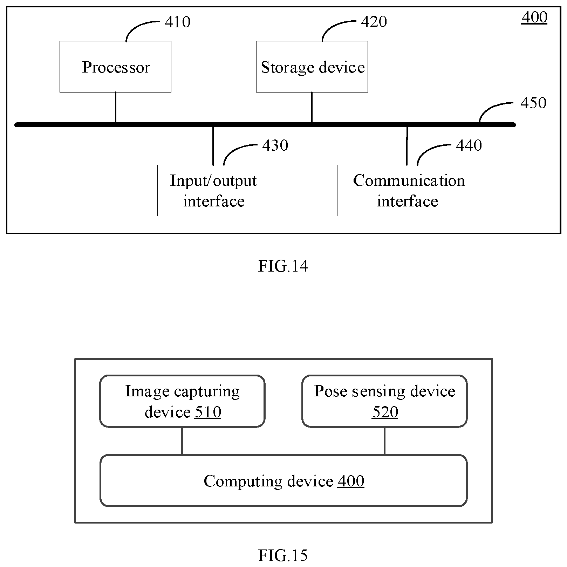

FIG. 14 shows a more specific hardware structure diagram of a computing device 400 according to an embodiment of the present application. The device may include a processor 410, a storage device 420, an input/output interface 430, a communication interface 440, and a bus 450. The processor 410, storage device 420, input/output interface 430, and communication interface 440 are connected to each other within the device via the bus 450 for communication.

The processor 410 may be implemented using a general-purpose central processing unit (CPU), a microprocessor, an application specific integrated circuit (ASIC), or one or more integrated circuits, etc., to execute related programs to implement the technical solutions provided by embodiments of the present disclosure. The processor 410 may also include a graphics card, which may be an Nvidia Titan X graphics card or a 10120Ti graphics card, etc.

The storage device 420 may be implemented as a read only memory (ROM), a random access memory (RAM), a static storage device, a dynamic storage device, etc. The storage device 420 may store an operating system and other applications. When implementing the technical solutions provided by embodiments of the present disclosure through software or firmware, related program codes are stored in the storage device 420 and invoked and executed by the processor 410.

The input/output interface 430 is used to connect an input/output module to implement information input and output. The input/output module may be configured as a component in the device (not shown in the figure) or may be externally connected to the device to provide corresponding functions. Input devices may include a keyboard, mouse, touch screen, microphone, various sensors, etc. Output devices may include a display, speaker, vibrator, indicator light, etc.

The communication interface 440 is used to connect a communication module (not shown in the figure) to implement communication interaction between this device and other devices. The communication module may implement communication via wired means (e.g., USB, network cable, etc.) or wireless means (e.g., mobile network, WIFI, Bluetooth, etc.).

The bus 450 includes a pathway for transmitting information between various components (e.g., processor 410, storage device 420, input/output interface 430, and communication interface 440) of the device.

It should be noted that although the above device only shows the processor 410, storage device 420, input/output interface 430, communication interface 440, and bus 450, in specific implementation, the device may also include other components necessary for normal operation. Furthermore, those skilled in the art can understand that the above device may also only contain components necessary to implement the solutions of the embodiments of the present disclosure, and does not have to contain all components shown in the figure.

Referring to FIG. 15, an embodiment of the present application further provides a medical assistance system. The system includes devices as follows.

An image capturing device 510 is configured to capture an image or a video frame of a target object.

A pose sensing device 520 is configured to detect a pose of the target object.

The computing device 400 that is from the aforementioned embodiments.

In some embodiments, the image or the video frame of the target object may be at least one of: the first image Img1 of the target object Obj in the aforementioned embodiments, the original video frame f0 of the target object Obj in the aforementioned embodiments, and the video frame f of the target object Obj in the aforementioned embodiments.

In some embodiments, the pose of the target object may be at least one of: the initial pose Pose0 of the target object Obj at the time when the first image Img1 is captured, the initial pose Pose0 of the target object Obj at the time when the original video frame f0 is captured, and the initial pose Pose0 of the target object Obj at the time when the video frame f is captured.

In some embodiments, the computing device may have a pre-trained neural network deployed thereon.

In some embodiments, the medical assistance system may be the robot surgical system 10 as shown in FIG. 1. The computing device may be disposed at at least one of: the patient side robot 101, the surgeon console 102, the vision cart 103; or the computing device may be separately provided.

In some embodiments, at least one robotic arm of the patient side robot 101 is used to hold a surgical instrument, and at least another robotic arm is used to hold the image capturing device.

Embodiments of the present application also provide a computer-readable storage medium having a computer program stored thereon. When the program is executed by a processor, the method according to any embodiment of the present application is implemented.

The computer-readable medium include both permanent and non-permanent, removable and non-removable medium that may be implemented by any method or technology for storing information. The information may be computer-readable instructions, data structures, program modules, or other data. Examples of the computer storage medium include, but are not limited to, phase-change memory (PRAM), static random access memory (SRAM), dynamic random access memory (DRAM), other types of random access memory (RAM), read-only memory (ROM), electrically erasable programmable read-only memory (EEPROM), flash memory or other memory technology, read-only compact disc read-only memory (CD-ROM), digital versatile disc (DVD) or other optical storage, magnetic cassettes, magnetic tape, magnetic disk storage or other magnetic storage devices, or any other non-transitory medium that may be used to store information accessible by the computing device. As defined herein, the computer-readable medium does not include transitory medium, such as modulated data signals and carrier waves.

From the descriptions of the above implementations, it can be seen that those skilled in the art can clearly understand that embodiments of the present application may be implemented by means of software plus a necessary general hardware platform. Based on such understanding, an essential part or a part contributing to the prior art of the technical solutions of the embodiments of the present application may be embodied in a form of a software product. The computer software product may be stored in a storage medium, such as ROM/RAM, magnetic disks, optical discs, etc., and includes several instructions for causing the computing device (which may be a personal computer, a server, or a network device, etc.) to execute the methods described in various embodiments of the present application or parts of the embodiments.

The systems, apparatuses, modules, or units illustrated in the above embodiments may be specifically implemented by computer devices or entities, or by products having certain functions. A typical implementation device is a computer. The specific form of the computer may be a personal computer, a laptop computer, a cellular phone, a camera phone, a smartphone, a personal digital assistant, a medium player, a navigation device, an email device, a game console, a tablet computer, a wearable device, or any combination of these devices.

Various embodiments in this specification are described in a progressive manner. The same or similar parts between various embodiments may be referred to each other. Each embodiment focuses on differences from other embodiments. Especially for apparatus embodiments, since they are basically similar to method embodiments, the description is relatively simple. For relevant parts, reference may be made to the descriptions in the method embodiments. The apparatus embodiments described above are merely illustrative. The modules described as separate components may or may not be physically separate. The functions of the modules may be implemented in one or more software and/or hardware during the implementation of the solutions of the embodiments of the present application. Some or all of the modules may also be selected to achieve objectives of the embodiments of the present application according to actual needs. Those of ordinary skill in the art may understand and implement them without creative efforts.

The above descriptions are merely specific implementations of the embodiments of the present application. It should be noted that for those of ordinary skill in the art, various improvements and modifications may be made without departing from principles of the embodiments of the present disclosure. These improvements and modifications should also be considered as within a protection scope of the embodiments of the present disclosure.

## Claims

1. A tracking method, comprising:
acquiring a video frame of a first target object and initial pose information indicative of an initial pose of the first target object when the video frame is captured;
acquiring a three-dimensional model of the first target object;
acquiring prediction pose information of the first target object through a pre-trained neural network based on the video frame;
acquiring detection pose information of the first target object based on the initial pose information and the three-dimensional model;
matching the prediction pose information and the detection pose information to obtain a matching result; and
tracking the first target object according to the matching result.

2. The method according to claim 1, wherein the prediction pose information comprises a prediction pixel region, prediction keypoint information, and prediction orientation information of the first target object, and the detection pose information comprises a detection pixel region, detection keypoint information, and detection orientation information of the first target object; the matching the prediction pose information and the detection pose information comprises:
matching the prediction pixel region and the detection pixel region, matching the prediction keypoint information and the detection keypoint information, and matching the prediction orientation information and the detection orientation information.

3. The method according to claim 2, wherein the matching result comprises a pixel region matching result, a keypoint matching result, and an orientation information matching result, the tracking the first target object according to the matching result comprises:
determining a first confidence score that the prediction pixel region matches the detection pixel region according to the pixel region matching result;
determining a second confidence score that the prediction keypoint information matches the detection keypoint information according to the keypoint matching result;
determining a third confidence score that the prediction orientation information matches the detection orientation information according to the orientation information matching result; and
tracking the first target object according to the first confidence score, the second confidence score, and the third confidence score.

4. The method according to claim 2 or claim 3, wherein the acquiring detection pose information of the first target object based on the initial pose information and the three-dimensional model comprises:
projecting the three-dimensional model into a coordinate system of an image capturing device according to the initial pose to obtain the detection keypoint information and the detection orientation information of the first target object in the coordinate system of the image capturing device,
wherein the image capturing device is configured to capture the video frame; and
projecting the three-dimensional model onto a two-dimensional image plane corresponding to the video frame according to the initial pose to obtain the detection pixel region of the first target object within the two-dimensional image plane.

5. The method according to any one of claims 1 to 4, wherein the acquiring prediction pose information of the first target object through the pre-trained neural network comprises:
acquiring a bounding box of the first target object in the video frame; and
acquiring the prediction pose information of the first target object in the video frame based on the bounding box of the first target object in the video frame.

6. The method according to claim 5, wherein the neural network comprises a plurality of feature extraction layers configured to perform feature extraction on the target video frame;
the bounding box of the first target object in the video frame is acquired based on features output from at least one first feature extraction layer among the plurality of feature extraction layers;
the prediction pose information of the first target object in the video frame is acquired based on features output from at least one second feature extraction layer among the plurality of feature extraction layers;
wherein each second feature extraction layer is prior to each first feature extraction layer.

7. The method according to claim 6, wherein each of the feature extraction layers comprises an encoder and a decoder; an output end of an encoder of an i-th feature extraction layer is connected to an input end of an encoder of an (i+1)-th feature extraction layer and an input end of a decoder of the i-th feature extraction layer; an input end of the decoder of the i-th feature extraction layer is connected to an output end of a decoder of the (i+1)-th feature extraction layer; wherein i is a positive integer;
wherein each encoder is configured to perform down-sampling processing on features input the encoder, and each decoder is configured to perform up-sampling processing on features input the decoder.

8. The method according to any one of claims 5 to 7, wherein the acquiring the bounding box of the first target object in the video frame comprises: acquiring the bounding box of the first target object in the video frame based on the video frame and a tracking result of the first target object in a prior video frame.

9. The method according to any one of claims 5 to 8, wherein the acquiring the prediction pose information of the first target object in the video frame based on the bounding box of the first target object in the video frame comprises:
performing pooling processing on features within the bounding box of the first target object in the video frame to obtain the prediction pose information of the first target object.

10. The method according to claim 1, wherein the neural network is pre-trained based on sample images and label information of the sample images.

11. The method according to claim 10, wherein the sample images and the label information of the sample images are generated by:
acquiring a first image of a second target object and initial pose information indicative of an initial pose of the second target object when the first image is captured;
acquiring a three-dimensional model of the second target object;
projecting the three-dimensional model of the second target object onto the first image based on the initial pose information of the second target object to obtain a projected pixel region;
determining corrected pose information based on the initial pose information of the second target object and an overlap degree between the projected pixel region and a target pixel region of the second target object on the first image;
generating a second image of the second target object based on the first image, wherein the second image is regarded as one of the sample images; and
generating label information of the second target object in the second image based on the corrected pose informationing.

12. The method according to claim 11, wherein the first image is captured when the second target object is against a first preset background.

13. The method according to claim 11 or claim 12, wherein the generating the second image of the second target object based on the first image comprises:
replacing the first preset background in the first image with a second preset background to obtain the second image.

14. The method according to any one of claims 11 to 13, wherein the sample images are generated by a further operation comprising:
performing post-processing on the second image; the post-processing comprises at least one of: blurring processing, sharpening processing, noise reduction processing, and enhancement processing.

15. The method according to any one of claims 11 to 14, wherein the corrected pose information is indicative of a pose of the second target object when the overlap degree is maximized.

16. The method according to any one of claims 11 to 15, wherein the determining corrected pose information based on the initial pose information and the overlap degree between the projected pixel region and the target pixel region of the second target object on the first image, comprises:
optimizing the initial pose information using a preset pose optimization algorithm, and recalculating the overlap degree between the projected pixel region and the target pixel region; and
determining the corrected pose information in response to the overlap degree between the projected pixel region and the target pixel region being maximum.

17. The method according to any one of claims 11 to 16, wherein the overlap degree between the projected pixel region and the target pixel region is determined based on an intersection over union (IoU), a generalized intersection over union (GIoU), or a dice loss between the projected pixel region and the target pixel region.

18. The method according to any one of claims 11 to 17, wherein the label information of the sample images are generated by further operations comprising:
acquiring a mask of the second target object in the first image; and
determining the overlap degree between the mask of the second target object and the projected pixel region.

19. The method according to claim 18, wherein before determining the overlap degree between the mask of the second target object and the projected pixel region, the method further comprises:
performing smoothing processing on the mask.

20. The method according to any one of claims 1 to 19, wherein the first target object or the second target object comprises at least one surgical instrument; each surgical instrument is held by a robotic arm of a surgical robot, and the robotic arm is provided with one or more sensor configured to detect the initial pose of the surgical instrument held by the robotic arm; the video frame and the first image are captured by an image capturing device.

21. The method according to claim 20, wherein the three-dimensional model of the surgical instrument is associated with type and model information of the surgical instrument; the acquiring the three-dimensional model of the first target object or the second target object comprises:
acquiring a three-dimensional model of the surgical instrument held by the robotic arm according to the type and model information of the surgical instrument held by the robotic arm.

22. The method according to claim 21, further comprising:
determining the type model information of the surgical instrument held by each robotic arm based on an operation log of the surgical robot; or
determining the type model information of the surgical instrument held by each robotic arm based on user input.

23. The method according to claim 21, wherein the label information comprises the corrected pose information, and the type and model information of the surgical instrument.

24. A tracking apparatus, comprising:
a first acquisition module configured to acquire a video frame of a first target object and initial pose information indicative of an initial pose of the first target object when the video frame is captured, and to acquire a three-dimensional model of the first target object;
a second acquisition module configured to acquire prediction pose information of the first target object through a pre-trained neural network based on the video frame;
a third acquisition module configured to acquire detection pose information of the first target object based on the initial pose information of the first target object and the three-dimensional model;
a matching module configured to match the prediction pose information and the detection pose information to obtain a matching result; and
a tracking module configured to track the first target object according to the matching result.

25. The tracking apparatus according to claim 24, wherein the tracking apparatus further comprises:
a fourth acquisition module configured to acquire a first image of a second target object and initial pose information indicative of an initial pose of the second target object when the first image is captured, and to acquire a three-dimensional model of the second target object;
a projection module configured to project the three-dimensional model of the second target object onto the first image based on the initial pose information of the second target object to obtain a projected pixel region;
a correction module configured to determine corrected pose information based on the initial pose information and an overlap degree between the projected pixel region and a target pixel region of the second target object on the first image;
a generation module configured to generate a second image of the second target object based on the first image; and
a determination module configured to determine label information of the second target object in the second image based on the corrected pose information.

26. A computer-readable storage medium having a computer program stored thereon, which when executed by a processor causes the processor to implement the method according to any one of claims 1 to 23.

27. A computing device, comprising a memory, a processor, and a computer program stored on the memory and executable by the processor, wherein the processor, when executing the program, implements the method according to any one of claims 1 to 23.

28. A medical assistance system, wherein the system comprises:
an image capturing device configured to capture a video frame of a first target object;
a pose sensing device configured to detect an initial pose of the first target object when the video frame is captured; and
the computing device according to claim 27 having a pre-trained neural network deployed thereon.

29. The system according to claim 28, wherein,
the image capturing device is further configured to capture a first image of a second target object;
the pose sensing device is further configured to detect an initial pose of the second target object when the first image is captured.

30. The system according to claim 28 or claim 29, wherein the first target object or the second target object is a surgical instrument; the system further comprises:
a surgical robot comprising at least one robotic arm, each robotic arm being configured to hold a surgical instrument and provided with a pose sensing device.

31. The system according to claim 28 or claim 29, wherein the first target object or the second target object is a surgical instrument; the system further comprises:
a surgical robot comprising at least two robotic arms, the image capturing device and the surgical instrument each being held by a respective one of the at least two robotic arms, and at least the respective one of the at least two robotic arms which holds the surgical instrument being provided with the pose sensing device.
